Europäisches Patentamt

European Patent Office

Office européen des brevets

(19)

(11) Numéro de publication : **0 097 086**
**B1**

(12) **FASCICULE DE BREVET EUROPÉEN**

(45) Date de publication du fascicule du brevet :
30.12.86

(51) Int. Cl.⁴ : **A 61 B 6/04**, A 61 G 7/10

(21) Numéro de dépôt : **83401141.3**

(22) Date de dépôt : **03.06.83**

(54) **Table d'examen à guidage linéaire.**

(30) Priorité : **11.06.82 FR 8210241**

(43) Date de publication de la demande :
**28.12.83 Bulletin 83/52**

(45) Mention de la délivrance du brevet :
**30.12.86 Bulletin 86/52**

(84) Etats contractants désignés :
**DE GB IT NL**

(56) Documents cités :
**DE-A- 2 008 251**
**FR-A- 1 551 249**
**US-A- 3 810 263**
**US-A- 4 131 802**
**US-A- 4 262 204**

(73) Titulaire : **THOMSON-CSF**
**173, Boulevard Haussmann**
**F-75379 Paris Cedex 08 (FR)**

(72) Inventeur : **Chambron, Edmond**
**THOMSON-CSF SCPI 173, bld Haussmann**
**F-75379 Paris Cedex 08 (FR)**

(74) Mandataire : **Grynwald, Albert et al**
**THOMSON-CSF SCPI 19, avenue de Messine**
**F-75008 Paris (FR)**

EP 0 097 086 B1

## Description

L'invention concerne une table d'examen à guidage linéaire d'un panneau porte-patient, utilisable dans le domaine de la radiologie.

L'exploration radiologique d'un patient peut exiger que celui-ci soit soumis à des examens frontaux ou latéraux, de la tête aux pieds, et dans ce cas un déplacement longitudinal du panneau porte-patient facilite la poursuite de ces examens.

Pour les examens latéraux, il existe un problème, qui réside dans une discontinuité de l'absorption du rayonnement auquel est soumis le patient ; cette discontinuité d'absorption est créée par les bords longitudinaux du panneau porte-patient qui, afin de permettre son déplacement longitudinal, est muni sur ces bords de moyens de guidage. Ces moyens de guidage sont généralement métalliques et présentent au rayonnement une absorption très supérieure à celle du matériau dont est constitué le panneau porte-patient.

Une amélioration consiste à munir les bords du panneau, de moyens de guidage disposés le long d'une moitié seulement de la longueur de ce panneau ; néanmoins l'exploration radiologique latérale de la tête aux pieds du patient, bien qu'améliorée, n'est pas entièrement satisfaisante.

Un brevet, délivré aux Etats-Unis sous le N° US-A-4 262 204, décrit un dispositif radiologique dans lequel l'examen d'un patient est effectué après que ce dernier ait été transporté dans une zone d'examen, grâce à un panneau porte-patient déplaçable. Le panneau est déplacé selon son axe longitudinal par rapport à un socle dans lequel il est partiellement encastré, et son extrémité engagée dans la zone d'examen est en porte-à-faux. Seule la partie en porte-à-faux est dégagée du socle, et l'on constate que cet agencement ne permet pas l'exploration radiologique latérale, de la tête aux pieds du patient, dans des conditions satisfaisantes.

La présente invention concerne une table d'examen à guidage linéaire, permettant un déplacement longitudinal d'un panneau porte-patient, dont les bords longitudinaux présentent au rayonnement une absorption faible et homogène, sur toute la longueur du patient ; la table d'examen conforme à l'invention étant agencée pour ne pas nécessiter de pièces additionnelles sur les bords de ce panneau.

Selon l'invention, une table d'examen à guidage linéaire d'un panneau porte-patient, ledit panneau étant capable d'un déplacement longitudinal parallèlement à un premier axe, ladite table d'examen comportant un moyen support supportant ledit panneau, un premier rouleau, disposé transversalement à ce premier axe, sur lequel repose le panneau dont une première extrémité est en porte-à-faux dans une proportion variable en fonction du déplacement longitudinal de ce panneau, ce premier rouleau étant en position fixe par rapport au premier axe, est caractérisé en ce qu'une seconde extrémité dudit panneau, opposée à la première extrémité, est solidarisée audit moyen support, et en ce que les pieds dudit moyen support sont montés sur un socle de ladite table de manière à permettre le déplacement dudit moyen support parallèlement audit premier axe.

La description suivante, donnée à titre d'exemple non limitatif, permettra de mieux comprendre l'invention. Elle s'appuie pour plus de clarté sur des dessins annexés au nombre de quatre, parmi lesquels :

la figure 1 montre en perspective une première version d'une table d'examen selon l'invention, comportant un panneau porte-patient guidé linéairement ;

la figure 2 montre par une vue de côté, des éléments essentiels au guidage du panneau dans la première version de la table selon l'invention ;

la figure 3 montre en perspective le panneau porte-patient dans une seconde version de l'invention ;

la figure 4 montre schématiquement les mêmes éléments que ceux figurés dans la figure 3.

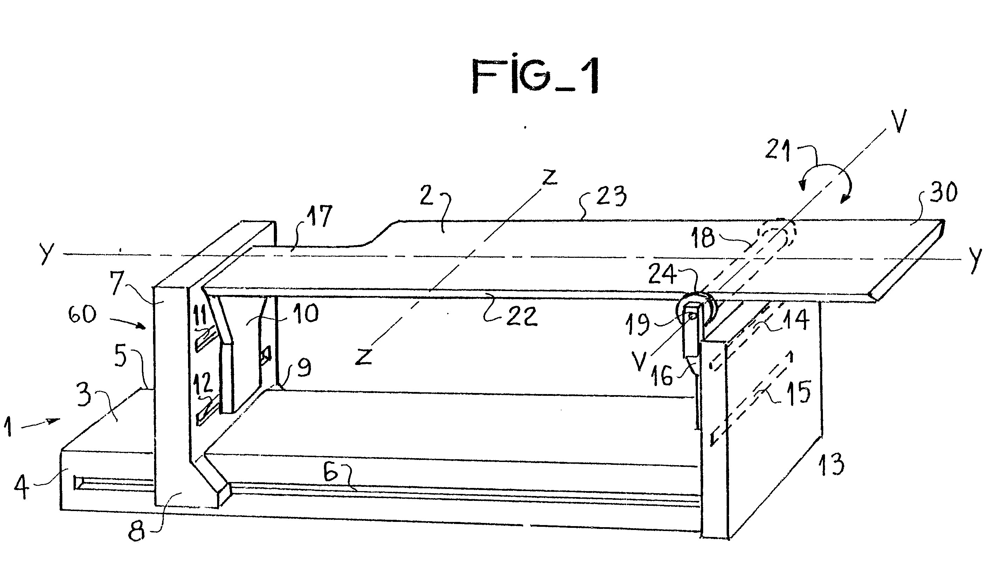

La figure 1 montre une table d'examen comportant notamment un panneau 2 porte-patient, déplaçable le long d'un premier axe longitudinal y-y ; cette table 1 peut également comporter des moyens nécessaires à l'examen d'un patient, ces moyens n'étant pas représentés pour une meilleure clarté de la description.

Un socle 3 servant de base à la table 1, comporte sur la longueur de chacun de ses côtés 4, 5 longitudinaux, une première gorge 6 visible sur le dessin uniquement d'un côté 4. Ces gorges 6 constituent un chemin de déplacement, parallèle à l'axe longitudinal y-y, pour un montant mobile 7 dont les pieds 8, 9 sont munis de moyens de roulement, non représentés, engagés dans ces gorges 6.

Le premier montant mobile 7 est associé à un second montant mobile 10 comportant des moyens de roulement non représentés, par lesquels ce second montant mobile coopère avec de secondes gorges 11, 12 constituant pour le second montant 10, un second chemin de déplacement parallèle à un second axe z-z transversal à l'axe longitudinal y-y.

La table 1 comporte également un troisième montant fixe 13, muni de troisièmes gorges 14, 15 représentées en traits pointillés sur la figure 1, et qui constituent un troisième chemin de déplacement pour un support de rouleau 16 mobile ; ce support de rouleau 16, mobile comporte des moyens de roulement, non représentés, engagés dans ces gorges 14, 15 de manière à permettre un déplacement de ce montant 16 parallèlement à l'axe transversal z-z.

Le panneau 2 est solidarisé à une première extrémité 17, au second montant mobile 10 qui, avec le premier montant mobile 7, constituent à ce panneau un moyen support 60.

Le panneau 2 comporte un autre support, sur lequel il repose, constitué dans l'exemple non limitatif décrit par un premier rouleau 18, représenté en traits pointillés sur la figure 1. Le rouleau 18 est disposé transversalement au panneau 2, selon un axe de rouleau v-v, parallèle à l'axe z-z ; il est fixé à ses extrémités au support de rouleau mobile 16 par des moyens de fixations 19, 20, dont seul le moyen 19 est visible sur la figure 1, de manière à être capable d'un mouvement de rotation autour de l'axe de rouleau v-v, selon la flèche 21.

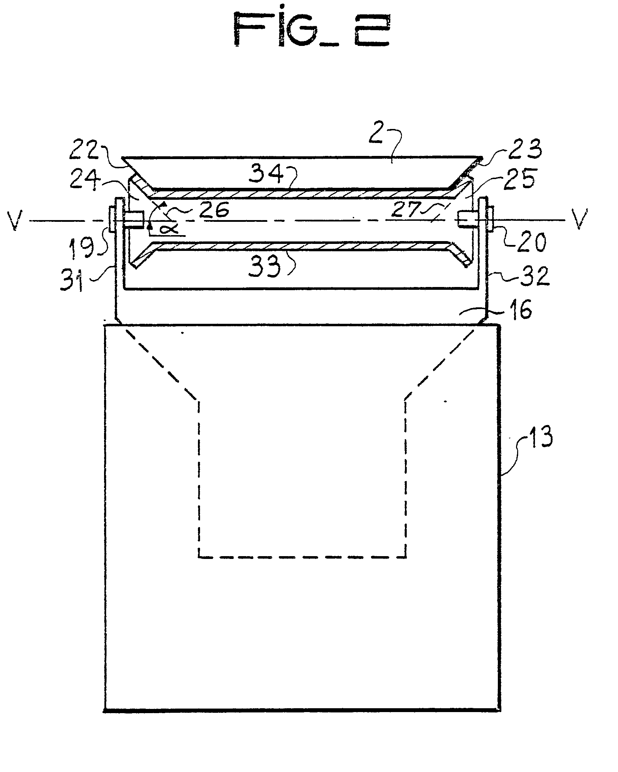

Cette disposition permet un premier déplacement longitudinal, manuel ou motorisé, du panneau 2, lequel est supporté par : d'une part le moyen support 60 constitué du premier et du second montant mobile 7, 10 guidés dans ce déplacement par les premières gorges 6 ; et d'autre part, supporté par le premier rouleau 18. Ce dernier, dans ce déplacement est mis en rotation tout en restant fixe par rapport au premier axe y-y ; le panneau 2 étant en porte-à-faux du côté de son extrémité 30, dans une proportion fonction de son déplacement. Le maintien latéral à ce niveau est obtenu par des premières butées latérales 24, 25 coopérant avec les flancs longitudinaux 22, 23 du panneau 2 ; l'une des butées 24 étant masquée sur la figure 1 mais visible sur la figure 2.

Un second déplacement du panneau 2, parallèlement à l'axe transversal z-z est obtenu grâce au deuxième montant 10 et au support de rouleau 16, coopérant pour ce déplacement avec respectivement les seconde et quatrième gorges 11, 12 et 14, 15.

Ceci constitue une caractéristique importante d'une table 1 d'examen 1 conforme à l'invention, qui permet de déplacer et de guider linéairement un panneau porte-patient 2, sans nécessiter d'adjoindre sur les bords de ce dernier des moyens de guidage et de déplacement ; le panneau 2 étant constitué sur toute la longueur d'un patient (non représenté), uniquement en matériau présentant au rayonnement utilisé pour l'examen, une absorption faible et homogène.

On montre sur la figure 2 une partie de la table 1 selon l'invention vue en bout, du côté comportant le premier rouleau 18 ; la représentation de cette table étant limitée au panneau 2 et, aux moyens nécessaires à réaliser le guidage, précédemment mentionnés.

Dans l'exemple non limitatif de la description, les butées latérales 24, 25 sont solidaires du rouleau 18 ; elles sont constituées par les extrémités de ce rouleau, et mises en rotation avec lui autour de l'axe de rouleau v-v.

Ces butées 24, 25 ont une forme conique dont la génératrice 26, 27 est parallèle aux bords des flancs longitudinaux 22, 23 du panneau 2 ; dans l'exemple non limitatif décrit cette génératrice 26, 27 présente par rapport à l'axe de rouleau v-v un angle α de 45°.

Le support de rouleau mobile 16, supporté par le troisième montant fixe 13, comporte deux bras 31, 32 entre lesquels est maintenu le premier rouleau 18 ; ce dernier, fixé ainsi qu'il a été précédemment expliqué par les moyens de fixation 19, 20 est capable d'un mouvement de rotation autour de l'axe de rouleau v-v. Cette rotation est obtenue lors du déplacement longitudinal du panneau qui repose sur ce rouleau 18. Le rouleau 18 est garni sur son pourtour d'un matériau souple 33 constitué, dans l'exemple non limitatif décrit, par du caoutchouc ; ce matériau souple 33 permettant de mieux répartir les pressions de contact entre le premier rouleau 18 et la face inférieure 34 du panneau 2.

Cette description montre qu'un panneau 2 porte-patient est déplaçable selon un premier axe longitudinal y-y ou selon un second axe z-z, transversal au premier. Un tel panneau 2 grâce à sa coopération avec des moyens tels que le premier rouleau 18 et les butées latérales 24, 25 n'exige pas sur ses côtés longitudinaux, d'apport de pièces additionnelles de guidage. Ceci, outre l'avantage présenté par rapport à l'absorption du rayonnement, permet à ce panneau 2 d'être beaucoup plus léger et plus maniable que les panneaux porte-patient utilisés dans les salles d'examen selon l'art antérieur.

On montre sur la figure 3 une autre version de l'invention dans laquelle le rouleau 18 coopère, pour supporter le panneau 2, avec une pluralité de seconds rouleaux, R1, R2, R3 constituant le moyen support 60.

Le panneau 2 est maintenu parallèle au premier axe y-y, dans son déplacement longitudinal, grâce à deux rouleaux guideurs 40, 41 coopérant avec une bande souple 46.

Les premiers et seconds rouleaux 18, R1, R2, R3 sont supportés par des montants de la table 1, non représentés ainsi que la table, pour une meilleure clarté de la description.

Le premier rouleau 18 est disposé transversalement au panneau 2, son axe de rotation ou axe de rouleau v-v étant parallèle au second axe z-z, ainsi que dans la première version de l'invention. Les seconds rouleaux R1, R2, R3, au nombre de trois sont disposés selon des axes (non représentés) parallèles au second axe z-z, autour desquels ils sont capables d'un mouvement de rotation lors du déplacement longitudinal du panneau 2, dans les mêmes conditions que le premier rouleau 18.

Ces premiers et seconds rouleaux 18, R1, R2, R3 sont répartis le long du premier axe y-y, sur une longueur L1 inférieure à la longueur L2 du panneau 2 ; cette répartition est telle que le panneau 2 dans son déplacement longitudinal, compris entre des positions extrêmes P1, P2, est toujours supporté par au moins trois de ces rouleaux 18, R1, R2, R3.

Ceci constitue un exemple non limitatif, un nombre N différent de ces rouleaux pouvant être répartis sur la course du panneau 2 ; au minimum deux rouleaux étant nécessaires pour supporter entièrement le panneau 2.

Une bande souple 46 constituée en un matériau peu absorbant du rayonnement X, comme par exemple du Mylar ayant quelques dixièmes de mm d'épaisseur, est solidaire à ses extrémités 47, 48, des extrémités 17, 30 du panneau 2, (Mylar étant un nom commercial pour « polytéréphtalate d'éthylène », qui est connu notamment pour ses caractéristiques de robustesse mécanique). Cette bande 46, se déplaçant en même temps que le panneau 2, est plaquée contre la face inférieure 34 du panneau 2 dont elle s'écarte, pour tourner autour des rouleaux guideurs 40, 41, au nombre de deux dans l'exemple non limitatif décrit.

Les rouleaux guideurs 40, 41 sont disposés selon des axes G, H, parallèles à l'axe transversal z-z, autour desquels ils sont capables d'une rotation selon les flèches 49, 50. Ces rouleaux guideurs 40, 41 sont situés à un niveau inférieur à celui des premier et seconds rouleaux supports 18, R1, R2, R3, et sont munis à leurs extrémités de secondes butées latérales 52, servant à guider et à maintenir latéralement les bords 53, 54 de la bande 46.

Ceci constitue une caractéristique importante de l'invention, qui permet de maintenir le panneau 2 parallèle au premier axe y-y dans son déplacement longitudinal ; ceci étant obtenu grâce à la coopération entre les secondes butées 52 et la bande souple 46, laquelle est fixée aux extrémités 17, 30 du panneau 2.

Dans l'exemple montré par la figure 3, où le panneau 2 est à une position extrême, son extrémité 30 coïncidant avec la position P1, on observe que :

d'une part, le panneau 2 est supporté par le premier rouleau 18 et deux seconds rouleaux R1, R2.

d'autre part, la bande 46, partant de la seconde extrémité 30 du panneau 2, est plaquée contre la face inférieure 34, jusqu'au franchissement du premier rouleau 18, et s'écarte de cette face inférieure 34 pour tourner autour d'un rouleau guideur 40, puis d'un second rouleau R1 ; par ce dernier elle est à nouveau plaquée contre la face inférieure 34, jusqu'au franchissement d'un autre second rouleau R2, après lequel elle tourne autour d'un autre rouleau guideur 41, pour rejoindre la première extrémité 17 du panneau 2.

Dans l'exemple non limitatif décrit, les deux rouleaux guideurs 40, 41, sont situés le long du premier axe y-y, à une distance D1 l'un de l'autre inférieure à une distance D2 ; cette distance D2 constituant entre les positions P1 et P2 de l'extrémité 17 du panneau 2, la longueur de la course de ce panneau. La distance D1 est centrée par rapport à une position P3 centrale du panneau 2, afin que dans les positions extrêmes P1, P2 occupées par celui-ci, les rouleaux guideurs 40, 41, ne dépassent pas les extrémités 17, 30 de ce panneau 2.

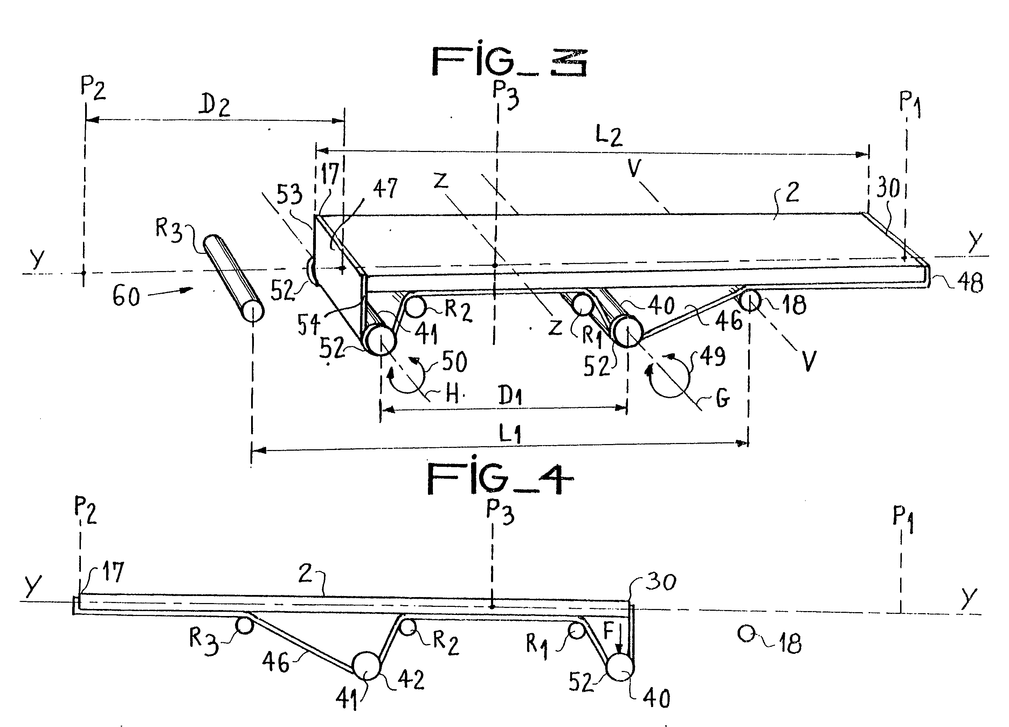

La figure 4 représente schématiquement le panneau 2 dans une autre position extrême de sa course longitudinale, déporté sur la gauche de la position centrale P3, où son extrémité 17 coïncide avec la position P2 ; ce panneau 2 est alors supporté par les trois seconds rouleaux R1, R2, R3, le premier rouleau 18 étant à son tour dégagé. La bande 46 occupe une position symétrique à celle précédemment décrite et tourne autour d'un rouleau guideur 40, pour rejoindre directement la seconde extrémité 30 du panneau 2.

Cette disposition évite un basculement du panneau 2 quand celui-ci se trouve dans une de ses positions extrêmes P1, P2, l'une de ses extrémités 17, 30 étant alors en porte-à-faux ; ce basculement est évité du fait que l'autre extrémité du panneau 2 est alors retenue par la bande 46, tournant autour d'un rouleau guideur 40, 41 qui se trouve alors au plus près de cette autre extrémité.

Des moyens permettant une tension de la bande 46 peuvent également être prévus ; cette tension pouvant être assurée, ainsi que dans l'exemple décrit, par au moins un rouleau guideur tel que le rouleau 40 par exemple, coopérant avec un moyen classique, non représenté, pour exercer sur ce rouleau guideur une force F appropriée.

Cette description montre que le panneau 2 est capable d'un déplacement longitudinal, parallèlement au premier axe y-y, dans lequel d'une part, il est supporté uniquement par des premier et seconds rouleaux 18, R1, R2, R3, et dans lequel d'autre part, il coopère avec des secondes butées latérales 52 par l'intermédiaire de la bande 46 souple.

Il est à remarquer qu'ainsi que dans la première version de l'invention, les côtés longitudinaux du panneau 2 ne nécessitent aucune pièce additionnelle de guidage.

**Revendications**

1. Table d'examen à guidage linéaire, d'un panneau (2) porte-patient, ledit panneau (2) étant capable d'un déplacement longitudinal parallèlement à un premier axe (y-y), ladite table d'examen (1) comportant un moyen support (60) supportant ledit panneau (2), un premier rouleau (18), disposé transversalement à ce premier axe, sur lequel repose le panneau (2) dont une première extrémité (30) est en porte-à-faux dans une proportion variable en fonction du déplacement longitudinal de ce panneau (2), le premier rouleau (18) étant en position fixe par rapport au premier axe (y-y), caractérisée en ce qu'une seconde extrémité (17) dudit panneau (2) opposée à la première extrémité (30) est solidarisée audit moyen support (60) et en ce que les pieds (8, 9) dudit moyen support (60) sont montés sur un socle (3) de ladite table d'examen de manière à permettre un déplacement dudit moyen support (60) parallèlement audit premier axe (y-y).

2. Table d'examen selon la revendication 1, caractérisée en ce qu'elle comporte en outre des premières butées latérales (24, 25) coopérant avec des flancs longitudinaux (22, 23) du panneau (2) pour guider latéralement ce panneau (2) dans son déplacement longitudinal.

3. Table d'examen selon la revendication 2, caractérisée en ce que les premières butées latérales (24, 25) sont solidaires du premier rouleau (18) et mises en rotation avec celui-ci autour d'un axe de rouleau (v-v).

4. Table d'examen selon l'une des revendications 2 ou 3, caractérisée en ce que le rouleau (18) et les premières butées latérales (24, 25) sont revêtues d'une matière souple (33), permettant de mieux répartir les pressions de contact avec le panneau (2).

5. Table d'examen selon la revendication 2, caractérisée en ce que les premières butées latérales (24, 25) ont une forme conique dont une génératrice (26, 27) est parallèle aux flancs longitudinaux (22, 23) du panneau (2).

6. Table d'examen selon la revendication précédente, caractérisée en ce que la génératrice (26, 27) des butées latérales (24, 25) présente par rapport au troisième axe (v-v) un angle ($\alpha$) de 45°.

7. Table d'examen selon la revendication 1, caractérisée en ce qu'un support de rouleau (16) supportant le premier rouleau (18), ainsi que le moyen support (60), sont déplaçables parallèlement à un second axe (z-z) transversal au premier axe (y-y), de manière à permettre un déplacement latéral du panneau (2).

8. Table d'examen selon la revendication 1, caractérisée en ce que le moyen support (60) est constitué par au moins une pluralité de seconds rouleaux (R1, R2, R3) coopérant avec le premier rouleau (18) pour supporter le panneau (2) dans son déplacement longitudinal entre deux positions extrêmes (P1, P2), dans lesquelles une des extrémités (17-30) du panneau (2) est en porte-à-faux, alors que l'extrémité opposée est maintenue en appui sur l'un des rouleaux (18, R1, R2, R3) grâce à une bande souple (46) retenue par des rouleaux guideurs (40, 41).

9. Table d'examen selon la revendication 8, caractérisée en ce que les extrémités (47, 48) de la bande souple (46) sont fixées aux extrémités (17-30) du panneau (2), et que cette bande souple (46) coopère avec les rouleaux guideurs (40, 41) pour maintenir le panneau (2) parallèle au premier axe (y-y), dans son déplacement longitudinal.

10. Table d'examen selon l'une des revendications 8, 9, caractérisée en ce que les rouleaux guideurs (40, 41) comportent de secondes butées latérales (52) coopérant avec des bords (53, 54) de la bande souple (46), pour maintenir le panneau (2) parallèle au premier axe (y-y) dans son déplacement longitudinal.

11. Table d'examen selon l'une des revendications 8 à 10, caractérisée en ce que la bande souple (46) est constituée en polytéréphtalate d'éthylène.

## Claims

1. Examination table having a linearly guided patient carrier panel (2), said panel (2) being capable of performing a longitudinal movement parallel to a first axis (y-y), said examination table (1) comprising a supporting means (60) supporting said panel (2), a first roller (18) arranged transversely to this first axis whereupon the panel (2) bears a first end (30) of which is cantilevered with a portion variable as a function of the longitudinal movement of this panel (2), said roller (18) being in a fixed position with respect to the first axis (y-y), characterized in that a second end (17) of said panel (2) opposed to the first end (30) is joined with said supporting means (60) and in that the legs (8, 9) of said supporting means (60) are mounted on a base (3) of said examination table in a manner to permit a movement of said supporting means (16) in parallel to said first axis (y-y).

2. Examination table according to claim 1, characterized in that it further comprises first lateral abutments (24, 25) cooperating with longitudinal flanges (22, 23) of the panel (2) for the lateral guiding of this panel (2) in its longitudinal movement.

3. Examination table according to claim 2, characterized in that the first lateral abutments (24, 25) are joined with the first roller (18) and rotated thereby about a roller axis (v-v).

4. Examination table according to claims 2 or 3, characterized in that the roller (18) and the first lateral abutments (24, 25) are covered with a yieldable material (33) permitting a better distribution of the contact pressures with the panel (2).

5. Examination table according to claim 2, characterized in that the first lateral abutments (24, 25) are of a conical shape one generatrix (26, 27) of which is parallel to the longitudinal flanges (22, 23) of the panel (2).

6. Examination table according to the preceding claim, characterized in that the generatrix (26, 27) of the lateral abutments (24, 25) forms an angle ($\alpha$) of 45° with the third axis (v-v).

7. Examination table according to claim 1, characterized in that a roller (16) bearing supporting the first roller (18) as well as the supporting means (60) are movable in parallel to a second axis (z-z) transversely to the first axis (y-y) in a manner to permit a lateral movement of the panel (2).

8. Examination table according to claim 1, characterized in that the support means (60) is constituted of at least one plurality of second rollers (R1, R2, R3) cooperating with the first roller (18) for supporting the panel (2) in its longitudinal movement between two end positions (P1, P2) wherein one of the ends (17-30) of the panel (2) is cantilevered, the opposed end being maintained in bearing relationship on one of the rollers (18, R1, R2, R3) by a flexible strip (46) retained by guide rollers (40, 41).

9. Examination table according to claim 8, characterized in that the ends (47, 48) of the flexible strip (46) are fixed to the ends (17-30) of the panel (2), and in that this flexible strip (46) cooperates with the guide rollers (40, 41) to maintain the panel (2) parallel to the first axis (y-y) during its longitudinal movement.

10. Examination table according to any of claims 8 and 9, characterized in that the guide rollers (40, 41) comprise second lateral abutments (52) cooperating with edges (53, 54) of the flexible strip (46) to maintain the panel (2) parallel to the first axis (y-y) during its longitudinal movement.

11. Examination table according to any of claims 8 to 10, characterized in that the flexible strip (46) is formed of polyethylene terephtalate.

**Patentansprüche**

1. Untersuchungstisch mit linearer Führung einer Patientenauflageplatte (2), die eine Längsverschiebung parallel zu einer ersten Achse (y-y) ausführen kann, wobei der Untersuchungstisch (1) eine Trägereinrichtung (60) umfaßt, welche die genannte Platte (2) lagert, mit einer ersten Rolle (18), die transversal zu dieser ersten Achse angeordnet ist, worauf die Platte (2) ruht, wovon ein erstes Ende (30) freitragend in einem Ausmaße ist, welches in Abhängigkeit von der Längsverschiebung dieser Platte (2) variiert, wobei die erste Rolle (18) sich in fester Stellung in bezug auf die erste Achse (y-y) befindet, dadurch gekennzeichnet, daß ein zweites Ende (17) der genannten Platte (2), welches dem ersten Ende (30) gegenüberliegt, fest mit der genannten Trägereinrichtung (60) verbunden ist, und daß die Füße (8, 9) der genannten Trägereinrichtung (60) auf einem Sockel (3) des genannten Untersuchungstisches derart angebracht sind, daß eine Verschiebung der genannten Trägereinrichtung (60) parallel zu der genannten ersten Achse (y-y) ermöglicht wird.

2. Untersuchungstisch nach Anspruch 1, dadurch gekennzeichnet, daß er ferner erste Seitenanschläge (24, 25) aufweist, die mit Längsflanken (22, 23) der Platte (2) zusammenwirken, um diese Platte (2) bei ihrer Längsbewegung seitlich zu führen.

3. Untersuchungstisch nach Anspruch 2, dadurch gekennzeichnet, daß die ersten Seitenanschläge (24, 25) fest mit der ersten Rolle (18) verbunden sind und mit dieser um eine erste Rollenachse (v-v) in Drehung versetzt werden.

4. Untersuchungstisch nach einem der Ansprüche 2 und 3, dadurch gekennzeichnet, daß die Rolle (18) und die ersten Seitenanschläge (24, 25) mit einem nachgiebigen Material (33) beschichtet sind, welches eine bessere Verteilung der Kontaktdrücke an der Platte (2) gestattet.

5. Untersuchungstisch nach Anspruch 2, dadurch gekennzeichnet, daß die ersten Seitenanschläge (24, 25) eine Kegelform aufweisen, wovon eine Mantellinie (26, 27) parallel zu den Längsflanken (22, 23) der Platte (2) ist.

6. Untersuchungstisch nach dem vorstehenden Anspruch, dadurch gekennzeichnet, daß die Mantellinie (26, 27) der Seitenanschläge (24, 25) gegenüber der dritten Achse (v-v) einen Winkel (α) von 45° einnimmt.

7. Untersuchungstisch nach Anspruch 1, dadurch gekennzeichnet, daß eine Tragrolle (16), welche die erste Rolle (18) sowie die Trägereinrichtung (60) lagert, parallel zu einer zweiten Achse (z-z) quer zu der ersten Achse (y-y) verschiebbar ist, so daß eine seitliche Verschiebung der Platte (2) ermöglicht wird.

8. Untersuchungstisch nach Anspruch 1, dadurch gekennzeichnet, daß die Trägereinrichtung (60) durch wenigstens eine Mehrzahl von zweiten Rollen (R1, R2, R3) gebildet ist, welche mit der ersten Rolle (18) zusammenwirken, um die Platte (2) bei ihrer Längsbewegung zwischen zwei Extremstellungen (P1, P2) zu lagern, in welchen eines der Enden (17-30) der Platte (2) freitragend ist, während das gegenüberliegende Ende in Abstützung an einer der Rollen (18, R1, R2, R3) durch einen flexiblen Streifen (46) gehalten ist, welcher durch Führungsrollen (40, 41) festgehalten wird.

9. Untersuchungstisch nach Anspruch 8, dadurch gekennzeichnet, daß die Enden (47, 48) des flexiblen Streifens (46) an den Enden (17-30) der Platte (2) befestigt sind und daß dieser flexible Streifen (46) mit den Führungsrollen (40, 41) zusammenwirkt, um die Platte (2) bei ihrer Längsbewegung parallel zu der ersten Achse (y-y) zu halten.

10. Untersuchungstisch nach einem der Ansprüche 8 und 9, dadurch gekennzeichnet, daß die Führungsrollen (40, 41) zweite Seitenanschläge (52) umfassen, die mit Rändern (53, 54) des flexiblen Streifens (46) zusammenwirken, um die Platte (2) bei ihrer Längsbewegung parallel zu der ersten Achse (y-y) zu halten.

11. Untersuchungstisch nach einem der Ansprüche 8 bis 10, dadurch gekennzeichnet, daß der flexible Streifen (46) aus Polyäthylenterephtalat besteht.

FIG_1

# FIG_2

FIG_3

FIG_4